# FASCICULE DE BREVET EUROPEEN

(11) **EP 2 538 932 B1**
(45) Date de publication et mention de la délivrance du brevet: **22.11.2017**
(21) Numéro de dépôt: 11713497.3
(22) Date de dépôt: 28.02.2011
(51) Int. Cl.: A61K 31/00, A61K 36/54, A61K 36/53, A61P 17/12, A61P 35/00, A61K 8/97, A61Q 19/00, A61K 31/05, A61K 31/045

(54) **UTILISATION D'HUILE ESSENTIELLE D'ORIGAN OU DE BOIS DE ROSE DANS LE TRAITEMENT DES KERATOSES ACTINIQUES**
VERWENDUNG EINES ÄTHERISCHEN ÖLS AUS OREGANO ODER ROSENHOLZ BEI DER BEHANDLUNG VON AKTINISCHER KERATOSE
USE OF ESSENTIAL OIL OF OREGANO OR ROSEWOOD IN THE TREATMENT OF ACTINIC KERATOSIS

(30) Priorité: 26.03.2010 US 282752 P; 26.02.2010 FR 1000801
(43) Date de publication de la demande: 02.01.2013
(73) Titulaire: L'Oréal, 75008 Paris (FR); Institut Curie, 75248 Paris Cedex 05 (FR); Centre National de la Recherche Scientifique, 75794 Paris Cedex 16 (FR)
(72) Inventeur: MARROT, Laurent, F-93190 Livry Gargan (FR); SOEUR, Jérémie, F-92340 Bourg La Reine (FR)
(74) Mandataire: Sellin, Carole
(86) Numéro de dépôt international: PCT/FR2011/050410
(87) Numéro de publication internationale: WO 2011/104489

(56) Documents cités:
- FR-A1- 2 830 198
- US-A1- 2008 213 410
- US-A1- 2008 233 218
- KAUR MANJINDER ET AL: "Skin cancer chemopreventive agent, alpha-santalol, induces apoptotic death of human epidermoid carcinoma A431 cells via caspase activation together with dissipation of mitochondrial membrane potential and cytochrome c release", CARCINOGENESIS, vol. 26, no. 2, février 2005 (2005-02), pages 369-380, XP002593567, OXFORD ISSN: 0143-3334 cité dans la demande
- DWIVEDI CHANDRADHAR ET AL: "Chemopreventive effects of alpha-santalol on skin tumor development in CD-1 and SENCAR mice.", CANCER EPIDEMIOLOGY BIOMARKERS AND PREVENTION, vol. 12, no. 2, février 2003 (2003-02), pages 151-156, XP002593568, ISSN: 1055-9965
- DWIVEDI C ET AL: "CHEMOPREVENTIVE EFFECTS OF SANDALWOOD OIL ON SKIN PAPILLOMAS IN MICE", EUROPEAN JOURNAL OF CANCER PREVENTION, vol. 6, no. 4, 1 août 1997 (1997-08-01), pages 399-401, XP001121309, LIPPINCOTT WILLIAMS & WILKINS, PHILADELPHIA, US ISSN: 0959-8278
- KOBA KOFFI ET AL: "In vitro cytotoxic activity of Cymbopogon citratus L. and Cymbopogon nardus L. essential oils from Togo", BANGLADESH JOURNAL OF PHARMACOLOGY, vol. 4, no. 1, 2009, pages 29-34, XP002593569, cité dans la demande
- KOBA K ET AL: "Chemical composition and in vitro cytotoxicactivity of essential oils from two tropical Lamiaceae:Aeollanthus pubescens Benth. and Ocimum gratissimum L", JOURNAL OF ESSENTIAL OIL-BEARING PLANTS, vol. 10, no. 1, 2007, pages 60-69, XP009136539, BHALLA, DEHRA DUN, IN ISSN: 0972-060X
- ANONYME: "Personal, Body, Care, Baby, Care, Cosmetics - Ingredients Used", , [Online] 11 mai 2009 (2009-05-11), pages 52-106, XP002593572, Earth, Origin, Natural, Organic Extrait de l'Internet: URL:http://www.earthorigin.co.za/ingredien ts.html> [extrait le 2010-07-22]
- ANONYMOUS: "Price List and Order Form", , [Online] 22 mars 2008 (2008-03-22), pages 1-6, XP002593573, The Victorian Garden Extrait de l'Internet: URL:http://web.archive.org/web/20080322083 726/http://www.thevictoriangarden.co.za/Pr iceOrder.html> [extrait le 2010-07-22]
- ANONYMOUS: "Welcome to the Victorian Garden of the 1800s...", , [Online] 25 juin 2010 (2010-06-25), pages 1-8, XP002593574, The Victorian Garden Organic Skincare Extrait de l'Internet: URL:http://webcache.googleusercontent.com/ search?q=cache:zOdC1BbW9d0J:www.thevictori angarden.co.za/AI_FacialProducts_pop-up.ht m+site:http://www.thevictoriangarden.co.za /AI_FacialProducts_pop-up.htm&cd=1&hl=en&c t=clnk&gl=de&tbo=1> [extrait le 2010-07-22]
- ANONYME: "Avocado Oil", , [Online] 14 juin 2008 (2008-06-14), pages 1-2, XP002593575, Nature's Gift Aromatherapy Products Extrait de l'Internet: URL:http://web.archive.org/web/20080614124 629/http://www.naturesgift.com/carrier_oil s/avocadoOil.htm> [extrait le 2010-07-22]
- DRBECKL2: "Skin Cancer Forum - wild oregano oil - skin cancer testimonies", , [Online] 26 mai 2008 (2008-05-26), XP002593576, Topicalinfo Extrait de l'Internet: URL:http://www.topicalinfo.org/forum/topic .asp?TOPIC_ID=308> [extrait le 2010-07-23]
- ANONYMOUS: "Testimonials", , [Online] 6 août 2007 (2007-08-06), pages 1-7, XP002593577, Hedd Wynn Essentials Extrait de l'Internet: URL:http://web.archive.org/web/20070806205 829/http://www.wildoiloforegano.com/index. php?page=testimonials> [extrait le 2010-07-23]
- ANONYMOUS: "FAQ", , [Online] 6 août 2007 (2007-08-06), pages 1-7, XP002593578, Hedd Wynn Essentials Extrait de l'Internet: URL:http://web.archive.org/web/20070806205 742/http://www.wildoiloforegano.com/index. php?page=faq> [extrait le 2010-07-23]
- TANG XIUWEI ET AL: "CP-31398 restores mutant p53 tumor suppressor function and inhibits UVB-induced skin carcinogenesis in mice", JOURNAL OF CLINICAL INVESTIGATION, vol. 117, no. 12, décembre 2007 (2007-12), pages 3753-3764, XP002593795, ISSN: 0021-9738
- QEINSPAHR JANINE ET AL: "Expression of p53 protein in actinic keratosis, adjacent, normal-appearing, and non-sun-exposed human skin", CANCER EPIDEMIOLOGY BIOMARKERS AND PREVENTION, vol. 6, no. 8, 1997, pages 583-587, XP002593796, ISSN: 1055-9965
- ANONYME: "Welcome to Pure Healing Europe: Herpes Treatments and Warts Treatments", , [Online] 21 novembre 2009 (2009-11-21), pages 1-6, XP002593797, Pure Healing Organic Medicinal Treatments Extrait de l'Internet: URL:http://www.purehealing-eu.com/body-war ts/treatment-body-warts.htm> [extrait le 2010-07-20]
- J-P. ORTONNE: "From actinic keratosis to squamous cell carcinoma", BRITISH JOURNAL OF DERMATOLOGY, vol. 146, no. s61, 1 avril 2002 (2002-04-01), pages 20-23, XP055007540, ISSN: 0007-0963, DOI: 10.1046/j.1365-2133.146.s61.6.x cité dans la demande
- Anonymous: "Wild Oil of Oregano", , 17 janvier 2010 (2010-01-17), page 1, XP055007479, Extrait de l'Internet: URL:http://web.archive.org/web/20100117235 421/http://www.oiloforegano.com/oil-of-ore gano-research-article-3.html [extrait le 2011-09-19]
- Erhardt W, Götz E, Bödecker N, Seybold S: "Zander - Handwörterbuch der Pflanzennamen - 16. Auflage", 2000, Eugen Ulmer, Stuttgart, XP002659476, ISBN: 3-8001-5080-8 pages 556-557, page 556
- S Bouhdid ET AL: "Antibacterial and antioxidant activities of Origanum compactum essential oil", African Journal of Biotechnology, 16 mai 2008 (2008-05-16), pages 1563-1570, XP055007547, Extrait de l'Internet: URL:http://www.academicjournals.org/AJB/PD F/pdf2008/16May/Bouhdid et al.pdf [extrait le 2011-09-20]
- Chambers ET AL: "Essential Oils: Actinic Keratosis Spots On My Face", , 6 August 2008 (2008-08-06), pages 1-2, XP055162564, Retrieved from the Internet: URL:http://www.oil-testimonials.com/essent ial-oils/3058/actinic-keratosis-spots-on-m y-face [retrieved on 2015-01-15]
- Connie Higley ET AL: "Reference Guide for Essential Oils" In: "Reference Guide for Essential Oils", 1 January 2005 (2005-01-01), XP055162568, ISBN: 978-0-97-065832-6 page FP1-2, 245, 246, 486,
- Biomarkers & Prevention ET AL: "Epidemiology, Expression of p53 Protein in Actinic Keratosis, Adjacent, Normal- appearing, and Non-Sun-exposed Human Skin1", Cancer [, 1 January 1997 (1997-01-01), pages 583-587, XP055162620, Retrieved from the Internet: URL:http://cebp.aacrjournals.org/content/6 /8/583.full.pdf

## Description

La présente invention est dans le domaine de la prévention et du traitement ciblé des kératoses actiniques.
Il est également admis que des expositions solaires chroniques induisent des lésions dermatologiques de types kératoses actiniques et un photovieillissement de la peau (Ortonne, J. P. 2002).
Au plan dermatologique, les kératoses apparaissent comme des zones de dysplasie épithéliale que l'on rencontre dans les régions du corps fréquemment exposées au soleil, et sont souvent associées au photo-vieillissement. La majorité de ces kératoses, régressent spontanément (Ortonne, J. P. 2002). De plus, les kératoses ont un aspect inesthétique qui peut amener les sujets atteints à demander leur traitement en vue de leur élimination, par souci esthétique.
Dans la peau, il a été montré que les kératoses actiniques étaient souvent constituées de kératinocytes porteurs de mutation dans le gène p53 (Taguchi, M. et al. 1994). Le gène codant pour la protéine p53 est couramment endommagé par les UV et, dans certains cas, subit des mutations, notamment des mutations CC vers TT. Les cellules ainsi mutées peuvent perdre la réponse spécifique aux UV contrôlée par la protéine p53 : la réparation est alors moins efficace et la présence de dégâts à l'ADN n'entraîne plus d'arrêt de prolifération : la réplication peut se dérouler en présence de lésions, augmentant ainsi le risque d'erreurs (mutations) et entraînant, à terme, une forte instabilité génétique. Comme les cellules mutées entrent moins facilement dans le processus d'apoptose, elles finissent par manifester un avantage de croissance par rapport aux cellules normales. Ce processus d'expansion clonale de cellules anormales crée des patches précancéreux dans les organes atteints
Pour étudier ces mécanismes, il est possible d'utiliser des kératinocytes portant des mutations dans le gène p53. C'est le cas de la lignée HaCat qui a été initialement décrite par Fusenig et al. (Fusenig, N. E. et al. 1998) et qui est aujourd'hui très fréquemment mise en oeuvre dans différents laboratoires de recherche en dermatologie. Ainsi par exemple, des cellules HaCat ont été intégrées dans un modèle organotypique de peau en présence de kératinocytes normaux : dans un tel système, il a été possible de mimer les effets promoteurs de l'exposition solaire en montrant la capacité des UVB à stimuler l'expansion clonale des cellules HaCat (Mudgil, A. V. et al. 2003) mutées en p53. Plus récemment, il a été rapporté que des cellules HaCat exposées aux UVA et réimplantées dans des souris pouvaient être à l'origine de tumeurs cutanées (Wischermann, K. et al. 2008). Il existe donc un besoin pour des produits induisant une cytotoxicité significativement plus intense dans des cellules HaCat par rapport à des kératinocytes humains normaux (ou à des lignées kératinocytaires non mutées en p53) qui seraient potentiellement capables de traiter les kératoses actiniques. C'est pour cette raison que les travaux décrits ci-après ont consisté à comparer et comprendre l'effet cytotoxique d'huiles essentielles sur des kératinocytes humains normaux, sur des kératinocytes HaCat (porteurs d'une mutation en p53) et sur des kératinocytes immortalisés issus de carcinome.
L'induction d'une mort cellulaire par apotpose, préférentiellement dans des cellules présentant des troubles de la prolifération et de la différenciation, tout en conservant l'intégrité des cellules saines constituerait un traitement efficace contre les lésions de type kératoses actiniques.
L'apoptose est un processus complexe qui peut avoir des origines diverses, mais il est aujourd'hui établi que la mitochondrie y joue un rôle important. Différents signaux moléculaires, comme par exemple l'équilibre Bcl2/Bax avec le possible concours de p53, sont capables de modifier la perméabilité de la membrane mitochondriale et de favoriser le relargage de cytochrome C dans le cytosol. Ce processus peut initier l'activation de la voie des caspases, enzymes en charge de la dégradation de certains composés intracellulaires, qui conduit de façon irréversible à l'apoptose (Gogvadze et al., 2008). Par ailleurs, il a aussi été montré que la perméabilisation de la membrane mitochondriale permettait le déversement de différentes molécules mitochondriales pro-oxydantes dans le cytosol, permettant l'induction de l'apoptose.

Les huiles essentielles (HE) et leurs produits dérivés, rencontrent actuellement un succès important auprès des consommateurs. Cet attrait tout particulier pour les produits d'origine naturelle (agroalimentaire, cosmétologie, pharmacologie...) ou issus du concept de naturalité, est la conséquence d'une demande générale de produits issus d'une production de type développement durable (chimie verte, agriculture bio). Cet intérêt pour la naturalité et ses produits de médecine douce (aromathérapie, phytothérapie) trouve également son origine dans les limites atteintes par des traitements de la médecine classique (résistances virales, bactériennes, traitement contre le cancer...). L'absence d'effets secondaires trop importants, dans le cadre de traitements aromathérapeutiques bien encadrés et raisonnés est un atout majeur pour l'utilisation des huiles essentielles.
Les huiles essentielles sont des produits volatiles complexes possédant une puissante odeur, caractéristique de la partie de la plante utilisée pour sa fabrication. Sur environ 800 000 espèces végétales répertoriées, seules les plantes aromatiques sont utilisées pour l'obtention d'huiles essentielles. Il s'agit de plantes possédant suffisamment de cellules synthétisant et sécrétant ces molécules aromatiques, soit environ 3000 plantes d'intérêt biologique. Environ 300 huiles essentielles représentent la majeure partie des huiles essentielles commercialisées.
Les huiles essentielles correspondent à un mélange complexe de métabolites secondaires (molécules non essentielles à la survie de la plante) synthétisés et sécrétés par des organes spécialisés : les poils glandulaires épidermiques, les poches et canaux glandulaires (schizogènes ou schizolysigènes). Ces métabolites secondaires sont représentés par une très grande diversité de molécules chimiques. Les plus courants sont les terpènes (mono-, sesqui- et diterpène : C10, C15 et C20 respectivement) et autres molécules aromatiques (molécules cycliques). Toutes les fonctions chimiques sont présentes dans les huiles essentielles: aldéhydes, cétones, alcools, peroxydes, lactones, éthers, esters...
Les huiles essentielles sont obtenues après hydro-distillation du matériel végétal de la plante aromatique. Aujourd'hui différentes méthodes d'extractions sont utilisées, parmi lesquelles on peut citer l'extraction au CO₂ supercritique et l'extraction par solvants.
Les huiles essentielles possèdent un potentiel cosmétique et thérapeutique reconnu, et sont principalement utilisées pour leurs activités bactéricides, virucides, antioxydantes, anti-inflammatoires, mais également pour leur caractère odorant, susceptible d'engendrer un sentiment de bien-être. Plusieurs modes d'utilisation des huiles essentielles sont possibles : inhalation, ingestion ou application cutanée. Les huiles essentielles pures ne sont quasiment jamais appliquées directement sur la peau, car elles sont souvent irritantes, mais diluées dans d'autres huiles végétales (huile d'olive, de tournesol...). Une application cutanée est réalisée dans le cadre de massages, de traitements locaux (infections) ou encore lors de l'utilisation de parfums (constituants majeurs).
Outre leurs intérêts cosmétiques, les huiles essentielles sont également utilisées dans le cadre de certains traitements antibiotiques, pour leur capacité à augmenter leur efficacité, ou encore pour lutter contre des infections des voies respiratoires. De la même façon, une proportion non négligeable (environ 35%) des patients européens atteints de cancer, dont les traitements classiques n'ont pu permettre la guérison, ont fait appel à des méthodes complémentaires et alternatives à la médecine classique (Molassiotis, A. et al. 2005), comme l'aromathérapie. Certaines études mettent en évidence des propriétés anticancéreuses et/ou anti-prolifératives des huiles essentielles vis-à-vis de certaines lignées cancéreuses. Ainsi, des travaux récents ont montré des activités potentiellement anti-carcinogènes ou antiprolifératives de certaines huiles essentielles sur des lignées de cellules leucémiques (Kumar, A. et al. 2008), (Verma, M. et al. 2008), lignées de cancer du colon (Sharma, P. R. et al. 2008), cellules de cancer du sein (Diaz, C. et al. 2008), cellules de mélanome (Loizzo, M. R. et al. 2008).

D'autre travaux ont montré également une activité anti-proliférative des certains composés isolés des huiles essentielles, comme l'alpha santalol sur une lignée de carcinome épidermoïde (Kaur, M et al. 2005), ou encore celle du terpinène-4-ol de l'huile essentielle de l'arbre à thé sur des cellules de mélanomes (Calcabrini, A. et al. 2004).
Concernant les kératinocytes, la cytotoxicité de certaines HE a été étudiée vis-à-vis de la lignée kératinocytaire HaCat mutée en p53 (Koba K. et al. 2009) ou de la lignée kératinocytaire SVK14 (Itharat, A. et al. 2004).
Sur le forum internet "Skin Cancer Forum - wild oregano oil- skin cancer testimonials" (http://www.topicalinfo.org/forum/topic.asp?TOPIC_ID=308), un internaute anonyme divulgue le traitement d'une lésion supposée être un cancer basocellulaire avec de l'huile essentielle d'origan sauvage. Sur le site internet publicitaire des produits Hedd Wyn Essentials, un internaute rapporte un prétendu effet thérapeutique d'une huile essentielle d'Origan sur un cancer de type SCC. Il n'est pas divulgué si ce cancer est issu d'une kératose, ni si les cellules sont mutées en p53.

L'invention concerne une composition comprenant une huile essentielle extraite de plantes de l'espèce *Origanum compactum* pour une utilisation chez un être humain dans le traitement ciblé de kératoses actiniques dépourvues de cellules tumorales, lesdites kératoses se caractérisant par des cellules porteuses de mutations dans le gène p53. L'invention concerne plus particulièrement l'huile essentielle extraite des différentes parties (racines, tiges, écorce, feuilles) des plantes *d'Origanum compactum.*
En effet, de façon surprenante, les présents inventeurs ont montré que des huiles essentielles de compositions moléculaires très différentes, à savoir l'huile essentielle d'Origan compact (*Origanum compactum,* famille des Lamiacées) et l'huile essentielle de Bois de rose (*Aniba rosaeodora*, famille des Lauracées), peuvent induire la mort cellulaire par apoptose de façon ciblée chez des kératinocytes humains cancéreux et précancéreux (mutés en p53) par rapport aux kératinocytes normaux.
Les compositions de ces deux huiles essentielles étant très différentes d'un point de vue chimique, et les constituants majoritaires également, les effets obtenus pour ces deux huiles essentielles peuvent donc être considérés comme représentatifs des effets obtenus avec toute huile essentielle ou leurs constituants.
Le traitement a en outre l'avantage de ne pas générer d'inflammation, l'apoptose n'étant pas un processus pro-inflammatoire.

L'invention concerne plus spécifiquement différentes compositions comprenant une huile essentielle d*'Origanum compactum,* dans la prévention ou le traitement des kératoses actiniques, préférentiellement pour l'être humain.

Dans le cadre de l'invention, les kératoses à traiter sont bénignes, dépourvues de cellules tumorales, notamment elles ne sont pas en phase de transformation. Dans cette situation en effet, un médecin ne recommanderait pas l'excision des kératoses pour des raisons thérapeutiques, étant donné le pourcentage important de kératoses qui disparaissent naturellement avec le temps, sans entrer en phase de transformation. Les kératoses considérées sont de préférence de petite taille, et ne présentent aucun danger de cancérisation à court ou moyen terme.
Par kératose (ou kératodermie ou hyperkératose) on entend une hyperplasie de la couche cornée (Stratum corneum) épidermique. Par kératose actinique ou kératose solaire, il est fait référence à une kératose induite par l'exposition au rayonnement solaire. Dans le cadre de la présente invention, des kératoses sont des kératoses actiniques. Par kératoses actiniques, on entend des lésions dépourvues de cellules tumorales.
Les kératoses et les kératoses actiniques considérées peuvent être constituées ou comprendre majoritairement des cellules mutées en p53.
De part leur aspect inesthétique, il existe en effet un fort besoin de prévention et / ou de traitement des kératoses, pour des raisons uniquement cosmétiques et non thérapeutiques.
Selon cet aspect, l'invention concerne une composition comprenant une huile essentielle d*'Origanum compactum,* pour une application cosmétique dans la prévention ou le traitement de kératoses. Les kératoses considérées sont notamment dépourvues de cellules tumorales.
Par prévention des kératoses, on entend notamment l'obtention d'un effet préventif tel que les kératoses ne se développent pas, ou celles qui sont déjà existantes ne se développent pas plus.
Par traitement des kératoses, on entend notamment un effet tel que les kératoses déjà existantes stoppent leur croissance et régressent, voire disparaissent entièrement.
La prévention ou le traitement des kératoses est particulièrement approprié dans le cas de peaux ayant été soumises à de fortes expositions aux UV ou des expositions répétées aux UV. Dans une telle application, la présente invention envisage notamment l'utilisation comme soin après soleil. La prévention peut concerner également l'application sur des peaux avant une exposition solaire, notamment avant de fortes expositions, afin de prévenir le développement de kératose, après vérification préalable toutefois de la non-phototoxicité des compositions ou huiles essentielles utilisées.
La prévention ou le traitement des kératoses, dans une application cosmétique selon l'invention, est également particulièrement approprié dans le cas de kératoses en phase de développement, c'est-à-dire que les kératoses sont déjà présentes sur la peau et qu'elles grossissent. Dans une telle application, l'utilisation selon l'invention permet de stopper la croissance des kératoses et de réduire leur taille, jusqu'à complète disparition éventuellement.
L'un des avantages majeurs des applications cosmétiques décrites réside dans l'action ciblée des huiles essentielles. En effet, l'huile essentielle présente, dans une fenêtre de concentration, une action ciblée à l'encontre des cellules précancéreuses (mutées en p53 mimant des cellules issus de kératose actinique), qui peuvent être hyperprolifératives ; les cellules normales sont quant à elles peu affectées par la cytotoxicité. Il est donc possible de traiter l'ensemble de la peau d'un individu sans aucun effet délétère pour les kératinocytes normaux. Cette qualité est particulièrement appréciable dans le cadre d'une application cosmétique. Il est à noter que les inventeurs sont en effet les premiers à avoir réalisé une étude comparée des impacts des huiles essentielles sur des kératinocytes mutés en p53 et sur des kératinocytes normaux. Aucune donnée relative à une telle comparaison de cytotoxicité n'avait été obtenue avant la présente invention. L'action ciblée des huiles essentielles et de leurs constituants n'avait donc jamais été mise en lumière avant l'invention. Au vu de ce qui précède, la présente invention permet donc une prévention ou un traitement ciblé des kératoses porteuses de mutations dans le gène p53, c'est-à-dire une moindre toxicité de ce traitement vis-à-vis des cellules normales, notamment des kératinocytes normaux, environnant la kératose.
Par cytotoxicité ciblée au sens de l'invention, on entend que la propriété de cytotoxicité est préférentiellement induite dans les cellules hyperprolifératives et/ou mutées en p53 (par exemple HaCat et A431) par rapport aux cellules non hyperprolifératives, non mutées en p53 (par exemple HEK001 et NHEK894), c'est-à-dire que les taux de toxicité sont au moins 1,5 fois plus élevés dans les cellules mutées que dans les cellules non mutées, de préférence au moins 2 fois plus élevés. De préférence, la viabilité des cellules non hyperprolifératives, non mutées reste de l'ordre de 40% au moins, voire de 50%, de préférence de 60%. Les inventeurs ont en effet mis en évidence l'existence de gammes de concentrations auxquelles une telle cytotoxicité ciblée, épargnant les cellules saines, pouvait être obtenue.
Du fait de l'action ciblée de l'huile essentielle d*'Origanum compactum,* les compositions pour le traitement cosmétique préventif selon l'invention sont sans danger pour les cellules saines non mutées en p53. Par conséquent, le traitement peut par exemple être renouvelé avant ou après chaque exposition au soleil.
Dans les compositions selon la présente invention, l'huile essentielle considérée constitue le principe actif de la composition, ou l'un des principes actifs, du fait que les inventeurs ont mis en évidence la cytotoxicité ciblée de l'huile essentielle d*'Origanum compactum* à l'encontre de kératinocytes mutés représentatifs de kératinocytes issus de kératoses. Selon un aspect de la présente divulgation, l'huile essentielle contient au moins un des constituants des huiles essentielles d*'Origanum compactum* ou *d'Aniba rosaeodora,* de préférence elle comprend au moins 10%, ou au moins 15 ou 20% en masse de linalol, de carvacrol ou de thymol. De préférence il s'agit d'une composition comprenant une huile essentielle ou un mélange d'huiles tel que l'huile essentielle ou le mélange comprenne lesdites proportions de linalol, de carvacrol ou de thymol. La divulgation concerne également des compositions où lesdits pourcentages correspondent à des pourcentages chromatographiques.
En effet, dans la section expérimentale, la cytotoxicité de deux huiles essentielles a été plus particulièrement étudiée : l'huile essentielle d'Origan compact (*Origanum compactum*, famille des Lamiacées) et l'huile essentielle de Bois de rose (*Aniba rosaeodora*, famille des Lauracées) sur des cellules épidermiques humaines : la lignée kératinocytaire HaCat (mutée en p53 et immortalisée spontanément), la lignée A431 (issue d'un carcinome spino-cellulaire, mutée en p53) ainsi que sur des kératinocytes épidermiques primaires normaux humains.
L'huile essentielle d'Origan compact se compose majoritairement de deux monophénols (terpénoïde) : le thymol et le carvacrol. Cette huile essentielle est préconisée dans le cadre de traitement de colites, de désordres pulmonaires. Elle permet de lutter contre la fatigue mais est surtout un puissant bactéricide à large spectre.
L'huile essentielle de Bois de rose se compose à 80% de linalol. Ses propriétés sont utilisées pour traiter les peaux irritées, fatiguées et ridées. Elle est également anti-infectieuse (moins puissante que l'huile essentielle d'Origan), immunostimulante et tend améliorer les comportements dépressifs. Elle est très faiblement irritante et faiblement toxique.
La présente invention concerne préférentiellement des compositions comprenant de l'huile essentielle d*'Origanum compactum*
Bien que l'activité cytotoxique d'un constituant isolé soit moindre que celle d'une huile essentielle complète, selon une autre mise en oeuvre de la divulgation, l'utilisation concerne plus spécifiquement au moins un constituant d'une huile essentielle, de préférence au moins un des constituants des huiles essentielles d*'Origanum compactum* ou *d'Aniba rosaeodora.*
Les différents constituants des huiles essentielles sont bien connus de l'homme du métier et peuvent être obtenus auprès des sociétés commercialisant lesdites huiles essentielles. Pour ce qui est des huiles essentielles d*'Origanum compactum* ou *d'Aniba rosaeodora,* l'exemple 1 de la présente demande fournit une caractérisation analytique des différents constituants détectés.

Il est tout particulièrement préféré, dans le cadre de la présente divulgation, que le constituant d'une huile essentielle dont il est fait usage ne soit pas l'oxyde de linalol, de préférence même pas un oxyde.
De préférence, le composant utilisé est un alcool ou un phénol, de manière plus particulièrement préférée un terpénol ou alcool terpénique, tel que le linalol, le terpinéol, le nérol, le géraniol, le citronellol, ou le menthanol, ou un terpénoïde tel que le citral, le menthol, le carvacrol ou le thymol, d'autres terpènes également envisagés sont les P-cymène, le terpinène, le myrcène et le caryophyllène béta. Des constituants tout particulièrement préférés sont le linalol, le carvacrol et le thymol.
Selon d'autres aspects des utilisations cosmétiques telles que décrites précédemment, le constituant est choisi parmi le 1,8-cinéole, le terpinolène, le linalol, l'alpha-terpinéol, le géraniol, l'alpha-copaène, l'alpha et le beta-selinène, le benzoate de benzyle, l'alphathujène, l'alpha pinène, le myrcène, l'alpha-phellandrène, l'alpha-terpinène, le paracymène, le beta-phellandrène, le gamma-terpinène, le thymol, le carvacrol et le beta-caryophyllène. Il s'agit en effet des constituants de l'une ou l'autre des deux huiles essentielles d'origan et de bois de rose, dont la proportion dans la partie volatile de l'huile est supérieure à 0,5% (en pourcentages chromatographiques).
Selon un autre aspect des applications cosmétiques, il est fait usage d'une huile essentielle comprenant au moins 0,5% en masse ou en pourcentages chromatographiques, de préférence au moins 1%, voire 5%, d'au moins l'un des composants suivants : le 1,8-cinéole, le terpinolène, le linalol, l'alpha-terpinéol, le géraniol, l'alpha-copaène, l'alpha et le beta-selinène, le benzoate de benzyle, l'alpha-thujène, l'alpha pinène, le myrcène, l'alpha-phellandrène, l'alpha-terpinène, le paracymène, le beta-phellandrène, le gamma-terpinène, le thymol, le carvacrol et le beta-caryophyllène. De préférence, il s'agit d'un pourcentage chromatographique, s'appliquant à la partie volatile de l'huile essentielle.
De manière préférée, il est fait usage dans les compositions de l'invention, d'au moins un constituant d'huile essentielle choisi parmi les composants suivants : le linalol, le thymol et le carvacrol. Il s'agit en effet, pour le linalol, du composé majoritaire de l'huile essentielle de bois de rose, et pour le thymol et le carvacrol, des deux composés majoritaires de l'huile essentielle d'origan.
Selon une mise en oeuvre, la divulgation concerne également l'utilisation d'une huile essentielle comprenant au moins l'un des constituants suivants : linalol, thymol et carvacrol. De préférence, au moins l'un de ces composants est présent en proportion supérieure à 0,5%, de préférence supérieure à 1%, 5% voire 10%, 15% ou 20% en masse. Alternativement, il peut s'agir de pourcentages chromatographiques. Une telle huile essentielle est par exemple l'huile essentielle de thym, de serpolet ou de sarriette (taux important de thymol ou carvacrol) ou l'huile essentielle de basilic, de thym (à linalol, Thymus vulgaris linaloliferum), de lavande ou de bois de Shiu (nommé également bois de Hô).
La présente divulgation concerne tout particulièrement l'utilisation cosmétique de compositions comprenant au moins 10%, de préférence au moins 15% ou au moins 20% de l'un des constituant suivants : linalol, carvacrol et thymol. D'autres compositions également envisagées sont telles que la teneur cumulée de linalol, carvacrol et thymol est supérieure à 10%, de préférence supérieure à 15%, voire supérieure à 20% en masse. Alternativement, il peut s'agir de pourcentages chromatographiques.
Selon une autre mise en oeuvre de la divulgation, le composant dont il est fait usage dans les applications cosmétiques, est choisi parmi l'alpha pinène, le camphène, le myrcène, l'alpha terpinène, le paracymène, le trans-ocimène, le gamma-terpinène, le linalol, le terpinen-4-ol, le beta-caryophyllène, l'alpha-humulène et l'oxyde de caryophyllène. Il s'agit en effet des composants qui sont communs aux deux huiles essentielles particulièrement préférées dans le cadre de la présente invention, à savoir l'huile essentielle d'origan et l'huile essentielle de bois de rose.
Selon une autre mise en oeuvre, la divulgation concerne également l'utilisation d'une huile essentielle comprenant au moins l'un des composants suivants : l'alpha pinène, le camphène, le myrcène, l'alpha terpinène, le paracymène, le trans-ocimène, le gamma-terpinène, le linalol, le terpinen-4-ol, le beta-caryophyllène, l'alpha-humulène et l'oxyde de caryophyllène. De préférence, au moins l'un des ces composants est présent en proportion supérieure à 0,5%, de préférence supérieure à 1%, 5% voire 10%, 15% ou 20% en masse. Alternativement, il peut s'agir de pourcentages chromatographiques.
Selon une autre mise en oeuvre de la divulgation, il est fait usage d'une huile essentielle, ou d'un constituant d'une huile essentielle qui soit capable de générer une situation pro-oxydante endogène, qui se produise spécifiquement dans des kératinocytes mutés en p53. Des huiles essentielles ayant cette propriété sont notamment l'huile essentielle d'origan compact et l'huile essentielle de bois de rose. Des résultats en ce sens sont présentés dans l'exemple 2 de la partie expérimentale. L'analyse moléculaire détaillée dans la section expérimentale montre que ces huiles essentielles provoquent une déstabilisation de la membrane mitochondriale et un relargage d'espèces réactives de l'oxygène, à l'origine de la cytotoxicité ciblée aux cellules mutées en p53.
Dans les exemples, sont aussi définis des tests simples pour mettre en évidence la génération d'une situation pro-oxydante, notamment par détection de la production de l'anion superoxyde mitochondrial.
L'huile essentielle selon l'invention, peut être extrait des racines, des tiges, de l'écorce ou des feuilles de ladite plante. Des techniques bien maîtrisées sont actuellement connues pour l'extraction des huiles essentielles, notamment l'extraction à froid qui consiste à soumettre la substance végétale à une forte pression à l'aide d'une presse hydraulique, l'entraînement vapeur, qui consiste à former de la vapeur d'eau qui traverse les végétaux et emporte avec elle les molécules aromatiques, et la distillation sèche. L'huile essentielle obtenue par distillation est une essence végétale modifiée par phénomène d'oxydation et d'hydrolyse. Une température maîtrisée et une basse pression sont essentielles pour conserver une qualité aromatique et une composition chimique les plus proches possible de l'essence végétale que l'on cherche à extraire. La plupart des huiles essentielles sont obtenues par distillation et entraînement à la vapeur d'eau (hydro-distillation).
D'autres techniques d'extraction sont bien connues et l'homme du métier saura, en fonction de la plante considérée, quelle technique est la plus appropriée. Il saura également, quelle technique est la plus appropriée en fonction de la partie de la plante qui va être utilisée pour l'extraction d'huile essentielle.
Il est bien entendu de préférence fait usage de techniques permettant la meilleure pureté et garantissant l'absence de solvants dans le produit extrait obtenu (huile essentielle ou constituant d'huile essentielle).
Dans le cadre des diverses compositions pour des applications cosmétiques selon la présente invention, il est de préférence fait usage d'une huile essentielle, en combinaison avec d'autres composés. Les autres composés peuvent notamment être des huiles végétales. Parmi les huiles végétales tout particulièrement préférées en combinaison avec les huiles essentielles selon l'invention, ou leurs constituants, on peut citer l'huile de pépins de raisin, l'huile d'amande douce, mais également l'huile de noisette, l'huile de macadamia, l'huile de tournesol et l'huile d'olive. Selon des mises en oeuvre particulières, l'huile essentielle peut être utilisée en combinaison avec un filtre solaire, ou en combinaison avec une solution hydratante ou une huile végétale, ou bien avec les deux.
Il peut aussi s'agir d'une combinaison d'au moins deux huiles essentielles distinctes, par exemple une huile essentielle extraite d*'Origanum compactum* et une huile essentielle extraite d'une plante de la famille des Lauracées.
Il peut également s'agir d'une combinaison comprenant une huile essentielle d'une plante avec un constituant de cette même huile essentielle, ce qui conduit à modifier les proportions naturelles des différents constituants de ladite huile essentielle, par exemple on peut utiliser de l'huile essentielle *d'Origanum compactum* en association avec du thymol ou du carvacrol.
Alternativement, il peut s'agir d'une combinaison comprenant une huile essentielle d'une plante avec un constituant provenant d'une autre huile essentielle, extraite d'une plante différente, notamment de l'huile essentielle *d'Origanum compactum* en association avec du linalol.

Il peut également s'agir de deux constituants distincts provenant d'huiles essentielles différentes, qui ne se trouvent pas naturellement ensemble au sein d'une quelconque huile essentielle, ou bien qui ne se trouvent pas naturellement dans ces proportions au sein d'une quelconque huile essentielle.
D'autres composés additionnels peuvent être choisis en fonction de la texture souhaitée et du mode d'application souhaité, pour les huiles essentielles ou leurs constituants dans les applications cosmétiques selon l'invention.
Par ailleurs, dans la présente invention, les inventeurs ont mis en évidence l'existence d'une gamme spécifique de concentration en huile essentielle, ou en constituant d'huile essentielle, pour laquelle la cytotoxicité est spécifiquement ciblée sur les cellules mutées en p53, par opposition aux cellules normales, et plus particulièrement encore sur les kératinocytes mutés en p53 par rapport aux kératinocytes normaux. Les utilisations cosmétiques envisagées dans le cadre de la présente invention sont de préférence des utilisations dans la gamme de concentration permettant une cytotoxicité ciblée aux cellules hyperprolifératives et/ou mutées en p53 et assurant une viabilité d'au moins 40%, voire d'au moins 50 ou 60% des cellules normales non mutées. La mise en oeuvre des protocoles détaillés dans les exemples 2 et 3 de la section expérimentale permet à l'homme du métier de déterminer les gammes de concentration adéquates pour toute huile essentielle ou l'un de ses composants.
Dans le cas d'une culture *in vitro,* les inventeurs ont montré (voir exemple 4) que lesdites gammes de concentration sont : de 0.0125% à 0.0175% pour l'huile essentielle d'Origan et de 0.035% à 0.045% pour l'huile essentielle de Bois de rose, afin d'obtenir une cytotoxicité ciblée dans les cellules mutées en p53.
En fonction de ces données, l'homme du métier saura adapter la composition selon l'invention pour obtenir une gamme de concentration comparable. Il peut notamment adapter la composition selon l'invention sous forme de patch pour garantir une mise en contact prolongée. Il est également envisageable de recommander des applications répétées, par exemple toutes les 24h durant une semaine ou durant un mois ou plus.
Les diverses utilisations cosmétiques au sens de l'invention s'entendent d'utilisations dans des conditions telles qu'elles induisent une cytotoxicité préférentielle dans les kératinocytes issus ou mimant une lésion de type kératose actinique, notamment mutés en p53, par rapport aux kératinocytes normaux ou non hyperprolifératifs.
Dans le cadre de cette invention, les utilisations mentionnées sont principalement envisagées pour des applications topiques, cutanées. C'est en effet le mode d'administration qui assure non seulement la meilleure efficacité, mais aussi un meilleur ciblage des cellules à traiter. Une formulation sous forme de patch est particulièrement préférée. D'autres modes d'administration sont toutefois envisageables, tels que l'ingestion et l'inhalation.
Une composition pour une utilisation dans le cadre de la présente invention comprend une concentration d'huile essentielle ou de constituant d'huile essentielle d'environ 0,03% à 0,15%, de préférence d'environ 0,03% à 0,1% d'huile essentielle d'Origan, ou d'environ 0,06% à 0,15% d'huile essentielle de bois de rose, pour une application cutanée.
Dans le cas des applications préventives, les compositions selon l'invention peuvent être formulées sous forme de lait, par exemple comme lait solaire, sous forme de pommade ou bien sous forme d'huile.
De préférence, l'huile essentielle choisie sera formulée en combinaison avec une huile végétale, notamment pour permettre sa dilution et diminuer ainsi son éventuel effet irritant. Par ailleurs, les inventeurs ayant mis en évidence le ciblage spécifique de l'effet cytotoxique des huiles essentielles ou de leurs constituants, notamment les constituants majoritaires, à l'encontre des cellules hyper prolifératives telles que les cellules mutées en p53, la présente divulgation concerne également toute utilisation cosmétique d'une huile essentielle ou de l'un de ses constituants, de préférence majoritaire, dans le traitement ou la prévention ciblé de zones inesthétiques de la peau dues à l'hyper-prolifération de cellules du derme ou de l'épiderme, par exemple dues à l'hyperprolifération de mélanocytes, notamment dans le cadre de naevi, ou bien des zones inesthétiques dans le cadre de cicatrices.
Un sujet susceptible d'être traité par les méthodes cosmétiques selon l'invention est un être humain, homme ou femme, quel que soit son âge. De préférence, il s'ait d'un adulte, mais l'invention n'est pas limitée aux adultes et comprend également le traitement d'adolescents, de préférence de plus de 15 ans, pour les diverses applications cosmétiques envisagées.
Selon un second aspect, la présente divulgation concerne également diverses méthodes cosmétiques, notamment des méthodes cosmétiques de prévention ou de traitement des kératoses actiniques, comprenant l'application sur la peau d'au moins une huile essentielle ou de l'un de ses constituants. Lesdites méthodes sont purement cosmétiques et ne visent qu'à modifier l'aspect esthétique de la peau. Lesdites kératoses peuvent être dépourvues de cellules tumorales.
Les diverses mises en oeuvre préférées dans le cadre de ces méthodes cosmétiques sont identiques aux mises en oeuvre détaillées plus haut concernant les diverses utilisations pour des applications cosmétiques.
Dans le cadre de la présente invention, les diverses applications précédemment décrites, préventives ou de traitement des kératoses sont également envisagées dans un but non seulement esthétique cosmétique, mais également à titre prophylactique, si l'évolution de la kératose vers une lésion pré-cancéreuse est soupçonnée ou envisagée, par exemple du fait de précédents familiaux ou chez le même sujet. Dans ce cas, il peut être envisagé de traiter une kératose pour des raisons non seulement cosmétiques mais également médicales, alors même que cette kératose n'est pas une tumeur et ne possède pas de cellule tumorale.
Dans ce cas, la présente invention est également dirigée vers des applications thérapeutiques des compositions précédemment décrites, comprenant une huile essentielle d*'Origanum compactum.*
Plus particulièrement, selon cet aspect, l'invention concerne une composition comprenant une huile essentielle d*'Origanum compactum,* pour une utilisation thérapeutique dans la prévention, ou le traitement ciblé de kératoses, de préférence chez les êtres humains. Lesdites kératoses s'entendent de kératoses actiniques, dépourvues de cellules tumorales, lesdites kératoses se caractérisant par des cellules porteuses de mutations dans le gène p53.
Bien que l'activité cytotoxique d'un constituant isolé soit moindre que celle d'une huile essentielle complète, la divulgation concerne notamment une composition comprenant un constituant de l'huile essentielle d*'Origanum compactum* ou d*'Aniba rosaeodora,* pour une utilisation thérapeutique dans la prévention, ou le traitement ciblé de kératoses. Ce constituant n'est de manière préférée par un oxyde, notamment pas un oxyde de linalol.
Notamment, comme déjà mentionné, la présente invention concerne plus spécialement des compositions comprenant une huile essentielle extraite de plantes d*'Origanum compactum.*
Comme pour les différentes applications déjà décrites, la composition comprenant l'huile essentielle d'*Origanum compactum,* est de préférence utilisée en combinaison avec d'autres composés, notamment des excipients thérapeutiquement acceptables ou des huiles végétales. Des composés additionnels particulièrement préférés ont déjà été décrits avec les autres aspects de l'invention et sont applicables aux utilisations thérapeutiques. Pour les diverses visées thérapeutiques de la présente invention, l'application topique de la composition est également envisagée comme dans les applications à visée purement cosmétique.
Selon la présente invention, les kératoses sont des kératoses actiniques.
Les gammes de concentrations préférées en huile essentielle ou en constituant d'huile essentielle, sont également celles qui assurent une cytotoxicité ciblée tout en préservant la viabilité des cellules saines non mutées, comme il a été décrit pour les autres aspects de l'invention.
Les compositions selon la présente invention sont de préférence telles qu'elles peuvent être utilisées pour induire une cytotoxicité préférentielle dans les kératinocytes issus ou mimant une lésion de type kératose actinique mutés en p53 par rapport aux kératinocytes normaux et/ou non hyperprolifératifs.

### Légende des figures :

**FIG.1** : La figure 1 représente la mesure de la viabilité cellulaire (test MTT) sur des cellules A431, HaCat et NHEK traitées 4h par l'HE d'Origan. Le trait double (=) correspond à la dose létale à 50% (DL50).
**FIG.2** : La figure 2 représente la mesure de la viabilité cellulaire (test MTT) sur des cellules A431, HaCat et NHEK traitées 4h par l'HE de Bois de rose. Le trait double (=) correspond à la dose létale à 50% (DL50).
**FIG.3** : La figure 3 représente la mesure de la viabilité cellulaire (test MTT) sur des cellules A431, HaCat et NHEK traitées 20h par l'HE d'Origan. Le trait double (=) correspond à la dose létale à 50% (DL50).
**FIG.4** : La figure 4 représente la mesure de la viabilité cellulaire (test MTT) sur des cellules A431, HaCat et NHEK traitées 20h par l'HE de Bois de rose. Le trait double (=) correspond à la dose létale à 50% (DL50).
**FIG.5** : La figure 5 représente la mesure de l'apoptose (test AV/PI), dans les cellules A431, HaCat et NHEK, après 12h de traitement par les HE d'Origan et de Bois de rose.
**FIG.6** : La figure 6 représente la mesure de la fluorescence (activité caspase) par cytomètrie en flux. Les cellules non marquées, les cellules marquées non traitées, et les cellules traitées (par HE) et marquées, sont étiquetées.
**FIG.7** : La figure 7 représente la mesure de la fluorescence (activité caspase) par microscopie à fluorescence.
**FIG.8** : La figure 8 illustre la quantification du nombre de cellules A431 et HEK 001 (kératinocytes non mutés) ayant subi une chute du potentiel membranaire mitochondrial après 6h de traitement par les HE d'Origan et de Bois de rose.
**FIG.9** : La figure 9 illustre la détection du stress oxydant induit après 4h d'incubation avec les HE d'Origan et de Bois de rose, dans les cellules A431, HaCat et NHEK 894, par la mesure (cytométrie en flux) de la fluorescence la sonde DHR 123.
**FIG.10** : La figure 10 représente la mesure de l'apoptose (test AV/PI), dans les cellules A431 et HEK001 (kératinocytes immortalisés non mutés), après 12h de traitement par les constituants d'huiles essentielles suivants : linalol, carvacrol et thymol.

### Section expérimentale :

### Exemple 1 : Etude analytique des huiles essentielles (HE) d'origan et de bois de rose.

Le choix d'étudier l'HE d'Origan a été déterminé par les travaux du laboratoire du Dr Averbeck qui ont montré une activité anti-génotoxique de cette HE chez la levure (Bakkali, F. et al. 2006). L'HE de Bois de rose a été choisie au regard de ses propriétés bénéfiques sur la peau.

Le présent exemple concerne la caractérisation de la partie volatile de 2 HE bio achetées dans le commerce :une huile essentielle de bois de rose et une huile essentielle d'origan. La méthodologie appliquée met en jeu, une analyse GC-MS, sur 2 colonnes de polarité différentes (colonne apolaire OV1 et colonne polaire VF WAX).

### Matériel et Méthode

### 1. Matériel

Les analyses ont été réalisées sur des appareils Agilent avec 2 types de colonnes :
- Colonne capillaire apolaire : Colonne capillaire HP-1 Longueur : 50m, diamètre interne : 0.2 mm, film : 0.33 µm.
- Colonne capillaire polaire : Colonne capillaire VF WAX, Longueur 60m, diamètre interne 0.25mm, film : 0.25µm.

Il est important de souligner que tous les pourcentages obtenus sont des pourcentages chromatographiques et non des pourcentages réels de composés présents dans la phase volatile. Pour quantifier exactement les constituants, il faudrait utiliser un étalon interne pour s'affranchir des variations dues à l'appareillage.

### 2. Identification des ingrédients à partir d'une chromatographie en phase gazeuse sur 2 colonnes de polarités différentes, couplée à un détecteur spectromètre de masse (GC/MS).

L'identification des molécules est réalisée à l'aide de bibliothèques de spectres de masse spécifiques aux parfums.

La sensibilité des appareils a permis de quantifier des composés pour lesquels le pourcentage relatif est supérieur ou égal à 0.001%.

La présentation des résultats et la quantification est réalisée, en GC / FID sur colonne apolaire, la quantification des constituants coélués est réalisée sur colonne polaire.

### 3. Echantillons analysés

- HE de bois de rose
- HE d'origan

### Résultats :

### Huile essentielle de Bois de rose :

A la vue des résultats, il apparaît que :
- L'on identifie avec certitude 99,12 % de l'aire totale du chromatogramme.
- 62 molécules ont été identifiées et quantifiées.
- Le tableau 1 de composition (en pourcentages chromatographiques) établi pour l'HE de bois de rose se trouve ci-dessous :

**Tableau 1 :**

| **BOIS DE ROSE** | **Composition en % GC** | **BOIS DE ROSE** | **Composition en % GC** |
|---|---|---|---|
| 6 METHYL 5 HEPTENE 2 ONE | 0,12 | MYRCENE | 0,05 |
| LINALOYL OXYDE = LIMETOL | 0,33 | HERBOXIDE (isomere) = DESOXYDE | 0,02 |
| BETA PINENE | 0,14 | | |
| BENZYL ALCOOL | 0,01 | GERANIAL | 0,05 |
| ALPHA TERPINENE | 0,02 | METHYL ANISOATE | 0,02 |
| PARACYMENE | 0,08 | GERANYL ACETATE | 0,02 |
| LIMONENE | 0,46 | ALPHA COPAENE | 1,15 |
| 1,8 CINEOLE | 0,57 | BETA ELEMENE | 0,13 |
| CIS BETA OCYMENE | 0,03 | ALPHA GURJUNENE | 0,15 |
| 5 DIMETHYL 2,2 TETRAHYDROFURANE = CTROXYDE | 0,05 | TRANS BETA CARYOPHYLLENE | 0,04 |
| | | ALPHA GUAIENE | 0,05 |
| TRANS BETA OCIMENE | 0,09 | ALPHA HUMULENE | 0,02 |
| GAMMA TERPINENE | 0,02 | ALLO AROMADENDRENE | 0,06 |
| CIS LINALOL OXYDE | 1,9 | GAMMA MUUROLENE | 0,04 |
| TRANS LINALOL OXYDE | 1,28 | SELINA4,11 DIENE | 0,14 |
| TERPINOLENE | 0,52 | BETA SELINENE | 0,82 |
| LINALOL | 78,93 | ALPHA SELINENE | 0,77 |
| HOTRIENOL | 0,2 | GAMMA CADINENE | 0,12 |
| ALPHA FENCHOL | 0,02 | DELTA CADINENE | 0,28 |
| MYRCENOL | 0,06 | TRANS NEROLIDOL | 0,27 |
| CIS OCIMENOL | 0,14 | SPATHULENOL | 0,11 |
| NEROL OXYDE | 0,03 | CARYOPHYLLENE OXYDE | 0,07 |
| TRANS OCIMENOL | 0,16 | alcool sesquit C15H24O HP1: 1637 | 0,23 |
| EPOXY LINALOL 1 = CIS LINALOL OXYDE (PYRANOÏDE) | 0,15 | alcool sesquit C15H24O HP1: 1646 | 0,15 |
| EPOXY LINALOL 2 = TRANS LINALOL OXYDE (PYRANOÏDE) | 0,25 | 7 EPI ALPHA EUDESMOL | 0,12 |
| | | alcool sesquit C15H24O HP1: 1689 | 0,27 |
| BORNEOL | 0,06 | alcool sesquit C15H24O HP1: 1697 | 0,29 |
| TERPINENE 4 OL | 0,2 | alcool sesquit C15H24O HP1: 1701 | 0,4 |
| ALPHA TERPINEOL | 4,52 | | |
| GAMMA TERPINEOL | 0,04 | BENZYL BENZOATE | 0,6 |
| NEROL | 0,38 | **TOTAL** (%) | **99,12** |
| NERAL | 0,04 | | |
| GERANIOL | 1,47 | | |

- Le composé majoritaire de l'HE est le linalol (78,93 %).
- Les composés présents en plus grande quantité dans l'HE, après le linalol, sont l'alpha terpinéol (4,52 %), l'oxyde de linalol cis (1,90 %), le géraniol (1,47 %), l'oxyde de linalol trans (1,28 %) et l'alpha copaène (1,15 %).
- Le composé minoritaire est l'alcool benzylique (0,01 %).

Les pourcentages massiques des composés majoritaires de l'huile essentielle de bois de rose sont les suivants :
- Linalol : 78,93%
- Terpinéol alpha : 4,91%.

### Huile essentielle d'origan :

A la vue des résultats, il apparaît que :
- L'on identifie avec certitude 97,69 % de l'aire totale du chromatogramme.
- 20 molécules ont été identifiées et quantifiées.
- Le tableau 2 de composition (en pourcentages chromatographiques) établi pour l'HE d'origan se trouve ci-dessous :

**Tableau 2 :**

| **ORIGAN** | **COMPOSITION EN % GC** | **ORIGAN** | COMPOSITION **EN % GC** |
|---|---|---|---|
| Thujène alpha | 0,87 | Terpinène gamma | 15,45 |
| Pinène alpha | 0,64 | Sabinène trans hydrate | 0,19 |
| Camphène | 0,12 | Linalol | 1,66 |
| 1 octen-3-ol | 0,23 | Terpinenol 4 | 0,61 |
| Octanone 3 | 0,17 | Thymol | 20,23 |
| Myrcène | 1,86 | Carvacrol | 38,61 |
| Phellandrène alpha | 0,23 | Caryophyllène beta | 1,88 |
| Terpinène alpha | 1,47 | Humulène alpha | 0,1 |
| Paracymène | 12,65 | Caryophyllène oxyde | 0,09 |
| Phellandrène beta | 0,56 | **TOTAL** (%) | **97,69** |
| Ocimène trans | 0,07 | | |

- Les composés majoritaires de l'HE sont le carvacrol (38,61 %), le thymol (20,23%), le terpinène gamma (15,45 %) et le paracymène (12,65 %).
- Le composé minoritaire est l'ocimène trans (0,07 %).
- Il existe 12 molécules communes entre l'HE d'origan et l'HE de bois de rose: l'alpha pinène, le camphène, le myrcène, l'alpha terpinène, le paracymène, l'ocimène trans, le terpinène gamma, le linalol, le terpinenol 4, le caryophyllène beta, l'alpha humulène, le caryophyllène oyde. Ces composés sont principalement des terpènes.

Les pourcentages massiques des composés majoritaires de l'huile essentielle d'origan sont les suivants :
- Paracymène : 12,27%
- Terpinène gamma : 16,10%,
- Thymol: 18,65%
- Carvacrol : 31,83%.

### Conclusions :

La composition chimique précise de chaque HE a été déterminée par analyse en chromatographie en phase gazeuse (voir tableaux 1 et 2). L'observation de la composition chimique de chaque HE révèle une différence qualitative très nette si l'on considère le nombre de composés majoritaires. Bien que l'HE de Bois de rose présente un plus grand nombre de molécules chimiques différentes (environ 62), elle possède un composé très majoritaire : le linalol, présent à plus de 78% de la fraction analysée. Au contraire, l'HE d'Origan, dans ses 20 molécules recensées, montre quatre molécules majoritaires : le carvacrol (38%), le thymol (20%), le terpinène gamma (15 %) et le paracymène (12 %). A ce titre, l'HE de Bois de rose est dite « mono-moléculaire » et l'HE d'Origan dite « polymoléculaire ». Il faut également remarquer que l'HE d'Origan est principalement composée de mono-phénol (carvacrol et thymol) alors que l'HE de Bois de rose est constituée majoritairement d'alcool terpénique (linalol).

### Exemple 2 :

Dans le présent travail, la cytotoxicité de deux HE a été plus particulièrement étudiée : l'HE d'Origan compact (*Origanum compactum*, famille des Lamiacées) et l'HE de Bois de rose (*Aniba rosaeodora*, famille des Lauracées) sur des cellules épidermiques humaines : la lignée kératinocytaire HaCat (mutée en p53 et immortalisée spontanément), la lignée A431 (issue d'un carcinome spino-cellulaire) ainsi que sur des kératinocytes épidermiques primaires normaux humains.

Ces trois modèles ont permis d'évaluer les effets biologiques potentiels de ces deux HE sur des cellules épidermiques normales, mutées en p53 (pouvant être assimilées comme précancéreuses) et cancéreuses, l'ensemble représentant dans une certaine mesure, les différentes situations entre peau saine et peau présentant un risque de cancer non mélanome et/ou peau issue de kératoses.

### Mesure de la viabilité cellulaire :

Les inventeurs ont comparé l'effet cytotoxique des huiles essentielles sur des kératinocytes normaux, mutés précancéreux (HaCat) et mutés cancéreux (A431).

La viabilité cellulaire est déterminée par le test MTT après un traitement de 4h et 20h par différentes concentrations en HE. Ce test (MTT) est basé sur l'activité enzymatique mitochondriale de la succinate déhydrogénase. Chaque point de viabilité est représenté par trois expériences indépendantes (NHEK : kératinocytes humains normaux « Normal Human Epidermal Keratinocyte »).

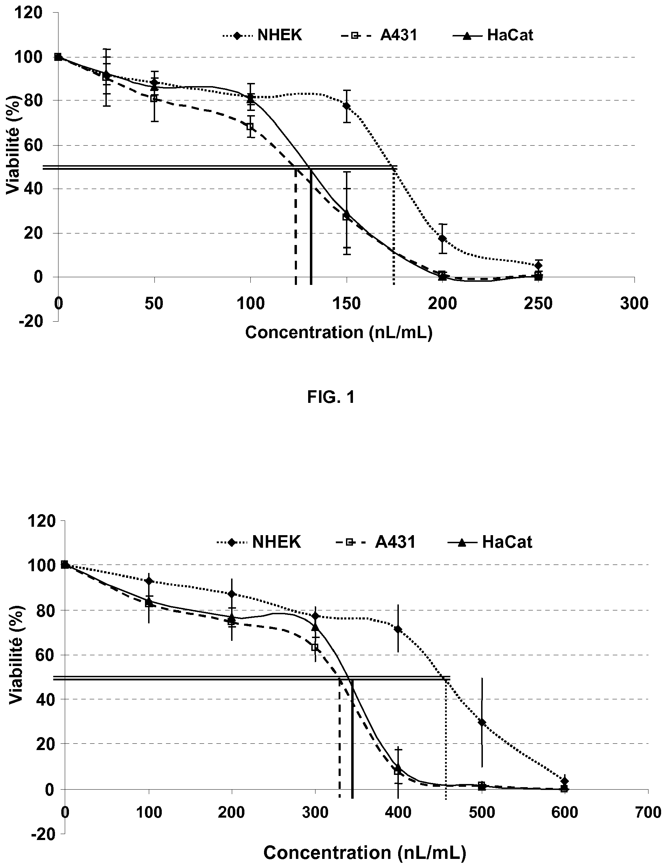

Les figures **1** et **2** indiquent que :
- l'HE d'Origan et plus cytotoxique que l'HE de Bois de rose, indépendamment du temps (comparaison entre les 4h et 20h de traitement) et de la lignée cellulaire utilisée.
- les cellules normales (NHEK) sont plus résistantes que les cellules cancéreuses (A431) et mutées précancéreuses (HaCat), après 4h de traitement.
- le profil de cytotoxicité est identique entre les cellules A431 et HaCat, après 4h de traitement (par les HE d'Origan et de Bois de rose).

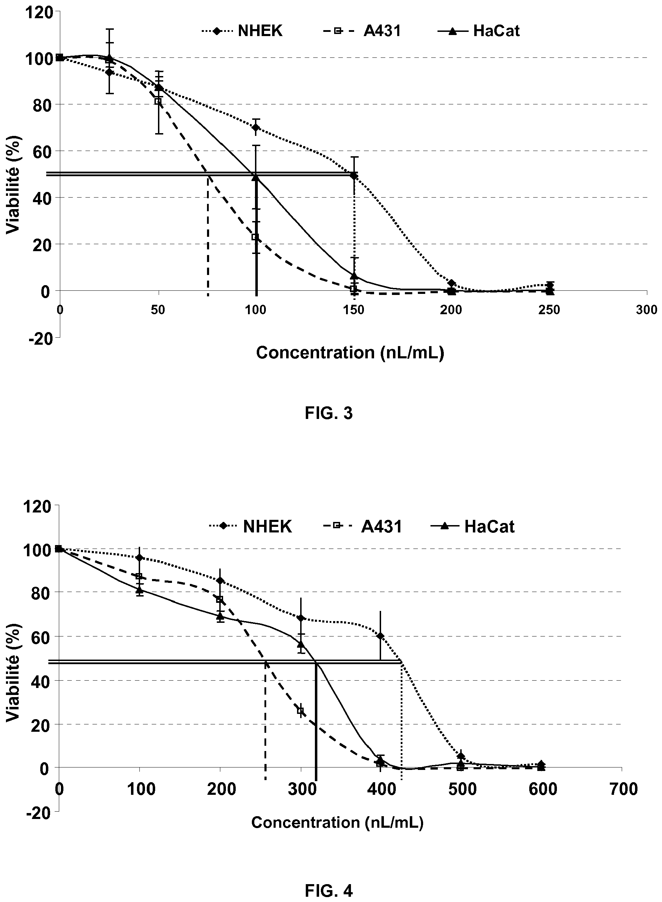

Les figures **3** et **4** confirment que cette différence de toxicité entre les cellules A431/HaCat et les cellules NHEK est conservée après 20h de traitement, bien qu'à ce temps une différence de sensibilité est observée entre les cellules HaCat et A431.
Ces résultats mettent en évidence une toxicité ciblée des HE pour les cellules cancéreuses et mutées précancéreuses par rapport aux cellules normales, à des concentrations de 150 nL/mL et 400 nL/mL d'HE d'Origan et de Bois de rose respectivement.

### Détection de l'apoptose :

L'apoptose se définit par l'induction d'événements biochimiques caractéristiques conduisant à une mort cellulaire programmée. Ces événements caractéristiques sont par exemple, la translocation d'un phospholipide membranaire particulier (phosphatidylserine) de la membrane plasmique interne ou encore l'activation de certaines protéases (caspases) impliquées dans ce processus de dégradation des protéines conduisant à la mort cellulaire.

Les résultats de viabilité cellulaire ont mis en évidence une concentration pour chaque HE, où est révélée la différence de sensibilité des cellules cancéreuses et mutées précancéreuses par rapport aux cellules normales: 150 nL/mL pour l'HE d'Origan et 400 nL/mL pour l'HE de Bois de rose. Ces concentrations seront utilisées dans le cadre d'un traitement de 16h (les 4h et 12h ont également été réalisés).

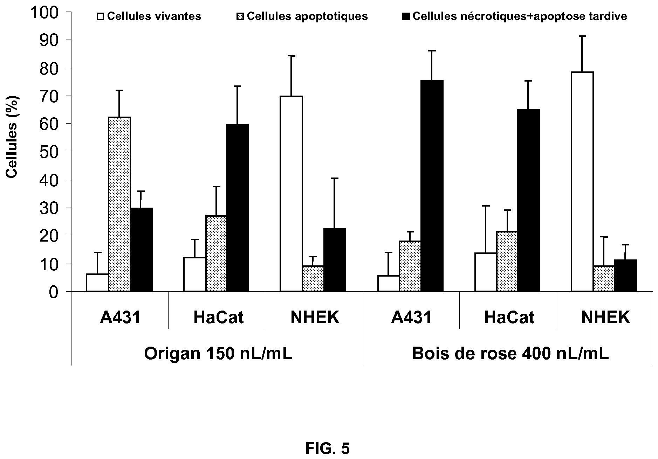

La figure **5** met en évidence après 12h de traitement:
- une viabilité significativement plus élevée des cellules normales (NHEK ; viabilité >70%) par rapport aux lignées cancéreuse A431 et mutée précancéreuse HaCat (taux de viabilité < 15% dans les lignées A431 et HaCat).
- une forte induction de l'apoptose dans la lignée cancéreuse A431 (plus de 60% de cellules annexine V positives).
- une apoptose tardive ou nécrose dans les cellules mutées précancéreuses HaCat. Ces résultats confirment une sensibilité plus élevée à la toxicité des HE des cellules mutées précancéreuses HaCat et cancéreuses A431 par rapport aux kératinocytes normaux NHEK. Ils mettent également en évidence une induction de mort cellulaire par apoptose.

Afin de confirmer l'induction d'une mort cellulaire par apoptose par les HE, un second test basé sur la détection de l'activité des caspases, une famille de protéines spécifiques du mécanisme de l'apoptose, est mis en oeuvre sur les cellules traitées par les HE. L'activation des caspases est un phénomène plus précoce que la translocation de la phosphatidylsérine détectée précédemment par l'annexine V. Le principe du test est basé sur l'utilisation d'un substrat de ces enzymes couplé à un fluorochrome. Les cellules en apoptose, où les caspases sont activées, dégraderont le substrat et émettent de la fluorescence. Les cellules non apoptotiques n'émettent pas de fluorescence. La mesure de fluorescence est réalisée par cytométrie en flux et microscopie à fluorescence, après 4h de traitement par les HE.

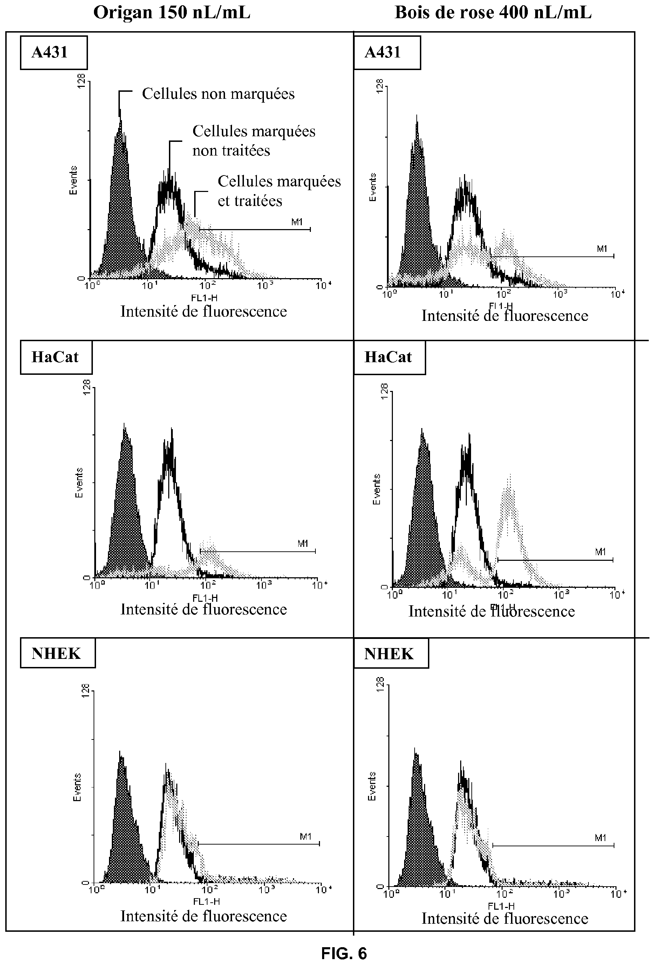

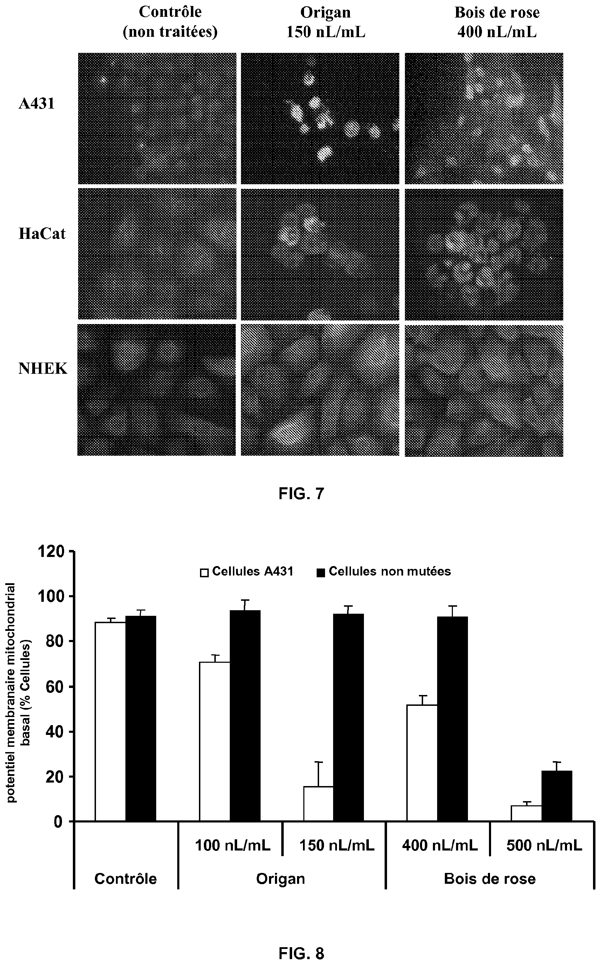

La figure **6** met en évidence un déplacement vers la droite (donc une augmentation de l'intensité de fluorescence) de la courbe représentant les cellules traitées par les HE par rapport à la courbe représentant les cellules marquées mais non traitées, dans les cellules A431 et HaCat uniquement. Cette augmentation de fluorescence, qui est confirmée par microscopie à fluorescence (figure **7**), traduit une activation des caspases et confirme :
- l'induction de l'apoptose dans les cellules cancéreuses, et HaCat par les deux HE, aux concentrations utilisées.
- que cette induction s'observe uniquement dans les cellules A431 et HaCat ; les cellules normales sont donc plus résistantes que les cellules cancéreuses et mutées précancéreuses pour un même traitement par les HE d'Origan et de Bois de rose.

### Effet des HE sur la mitochondrie

Après traitement par les HE d'origan et de bois de rose, l'analyse de l'intégrité de la membrane mitochondriale des kératinocytes mutés cancéreux A431 et des kératinocytes non mutés est réalisée par cytométrie de flux (suivi de la fluorescence liée à l'accumulation de TMRM dans la membrane mitochondriale intègre). On constate en figure **8** que dans le domaine de concentrations choisi, il y a bien un effet déstabilisant de la mitochondrie lié au traitement par les HE, mais cet effet est particulièrement marqué dans les cellules A431 par rapport aux kératinocytes non mutés. Cet impact sur la mitochondrie est très probablement associé au développement du processus apoptotique (cause et/ou conséquence).

### Génération intracellulaire d'espèces réactives de l'oxygène consécutive au traitement par les HE.

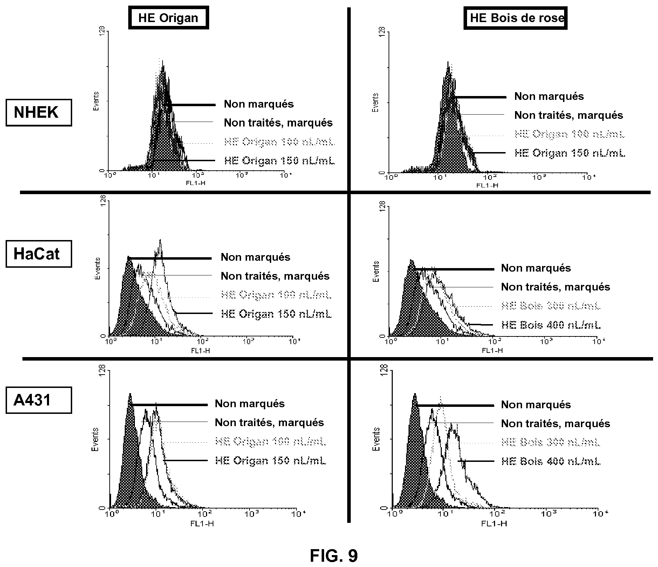

Une des conséquences de la déstabilisation mitochondriale est le relargage de molécules pro-oxydantes dans le cytosol, ce phénomène pouvant être détecté en cytométrie de flux par la sonde DHR123 qui fluoresce en cas de stress oxydant. La figure **9** montre l'évolution du nombre de cellules fluorescentes (donc sujettes à une génération d'espèces réactives de l'oxygène détectables par la DHR123) après différents traitements par les HE. On constate qu'aux concentrations utilisées, les kératinocytes normaux ne sont pas affectés alors qu'une augmentation dose-dépendante du statut pro-oxydant intracellulaire est décelable dans les cellules mutées HaCat ou A431. Ainsi, les HE sont capables de produire un stress oxydant significatif et spécifique dans les kératinocytes mutés précancéreux ou cancéreux, ce stress étant probablement fortement impliqué dans la cytotoxicité ciblée décrite par les figures 1 à 4.

### Conclusions

Les données présentées ici démontrent une sensibilité à la cytotoxicité des HE d'Origan et de Bois de rose significativement plus élevée chez des kératinocytes humains cancéreux et mutés précancéreux par rapport aux kératinocytes normaux.

L'analyse des mécanismes en jeu a permis de montrer que cette différence de sensibilité s'expliquait par l'induction d'une mort par apoptose dans les cellules cancéreuses et mutées précancéreuses, alors que les cellules normales présentaient des taux de viabilité cellulaire supérieurs à 70% pour une même concentration en HE.

L'analyse moléculaire montre que cette toxicité spécifique par les HE implique des modifications de l'intégrité mitochondriale et la génération d'un stress oxydant endogène. Il est donc envisageable de cibler des kératinocytes mutés pré-cancéreux (en particulier dans les kératoses ou dans des zones de la peau endommagées par les UV) par les HE et ainsi de les éliminer spécifiquement.

### Exemple 3 : Mesure de l'activité pro-apoptotique des composés isolés majoritaires des huiles essentielles d'Origan et de Bois de rose.

L'HE de Bois de rose est composée d'une molécule majoritaire (à plus de 80%) : le linalol (alcool terpénique). L'HE d'Origan Compact contient deux composés majoritaires (monoterpénol) : le carvacrol et le thymol, présent à 40% et 20%, respectivement.

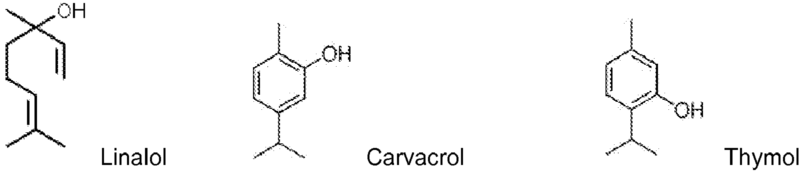

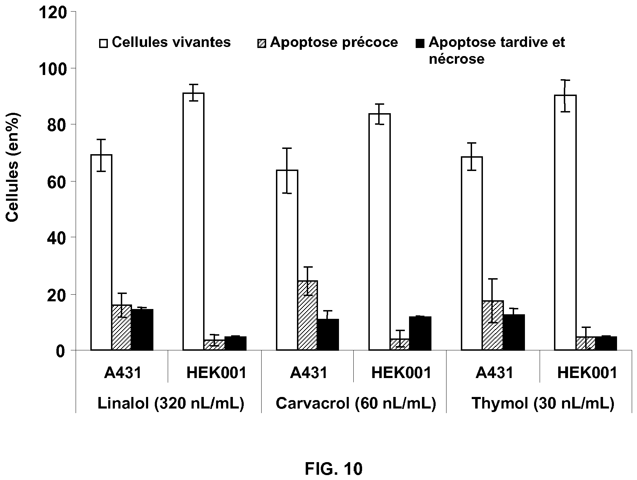

Ces trois molécules, ont été mises en incubation 12h avec des kératinocytes immortalisés non mutés (HEK 001) et des kératinocytes cancéreux mutés (A431). Les pourcentages de cellules en apoptose ont été mesurés par le test Annexine V/ lodure de propidium. Les concentrations des molécules utilisées sont égales à celles présentes lors des traitements par les HE. Exemple : la concentration de linalol utilisée ici, représente l'équivalent des 80% de linalol présent dans l'HE de Bois de rose. En effet, le linalol est utilisé à une concentration de 320 nL/mL (voir FIG.10), c'est-à-dire 80% de 400 nL/mL d'huile essentielle de Bois de rose qui est la concentration démontrée comme la plus toxique pour les cellules HaCaT et A431, tout en laissant un maximun de cellules NHEK et HEK 001 viables (voir Exemple 2, conclusions sur la section « mesure de la viabilité cellulaire » et FIG. 3 et 4 ainsi que la section « détection de l'apoptose » et FIG.5). Il en est de même pour le carvacrol et le thymol de l'HE d'Origan.

Les résultats d'apoptose sont présentés dans la figure **10****.**

Les résultats de cette expérience exprimés en % de cellules, vivantes, en apoptose ou en apoptose tardive et nécrose montrent:
- Une cytotoxicité et une activité pro-apoptotique plus faible des molécules majoritaires isolées par rapport à celle induite par les HE dans leur complexité (70% d'apoptose induite dans les cellules cancéreuses à dose équivalente d'HE d'Origan, soit 150 nL/mL). En effet, la FIG.5 fait apparaitre par exemple que plus de 60% des cellules A431 sont apoptotiques après un traitement par de l'HE d'origan à une concentration de 150 nL/mL. La FIG.10 au contraire montre qu'environ 25% seulement des cellules A431 sont apoptotiques après 12h de traitement avec le carvacrol à une concentration de 60nL/mL (correspondant à 40% de 150nL/mL) et environ 15% de cellules apoptotiques avec un traitement au thymol à 30 nL/mL (correspondant à 20% de 150nL/mL).
- Des taux de viabilité des cellules non mutées (HEK001) supérieurs à ceux observés dans les cellules mutées (A431). Le ciblage préférentiel des cellules mutées est donc conservé.

Cette expérience montre que la mort par apoptose ciblée aux cellules cancéreuses mutées est conservée qualitativement si l'on utilise les composés majoritaires du Bois de rose (Linalol) ou de l'Origan (Carvacrol et thymol). Par contre, l'efficacité cytotoxique est moindre qu'avec les HE dans leur complexité. Il existe donc vraisemblablement des synergies avec les autres constituants.

Ce point est confirmé par l'observation réalisée par les inventeurs que le mélange de plusieurs molécules isolées d'une même huile essentielle reproduit l'activité observée avec l'huile essentielle, activité supérieure à la simple addition des activités des molécules isolées prises séparément.

### Matériel et méthodes pour les exemples 2 et 3:

Trente six heures avant le traitement, les cellules sont distribuées en plaque 96 puits (5.10⁵ cellules/mL).

Solution de traitement des HE : les HE sont diluées au 1/10ème une première fois dans de l'éthanol 100%. Puis une seconde dilution est réalisée dans du milieu de culture afin d'obtenir les concentrations désirées.

La mesure de viabilité est réalisée à l'aide du test MTT (1-(4,5-Dimethylthiazol-2-yl)-3,5-diphenylformazan. Sigma-Aldrich). Brièvement, après le temps de traitement désiré, le milieu de traitement est remplacé par du milieu contenant la solution de MTT (0,25 µg/mL de concentration finale). La plaque 96 puits contenant les cellules est ensuite incubée 4h à 37°C avant d'éliminer la solution de MTT et d'ajouter 100 |jL de DMSO. La mesure d'absorbance est ensuite réalisée à 540 nm après avoir homogénéisé le précipité violet. La mesure du stress oxydant est réalisée à l'aide de la sonde fluorescente DHR 123 (Molecular Probe). Après 4h de traitement par les HE, le milieu est éliminé et les cellules sont récoltées (par trypsinisation), centrifugées puis rincées dans 4mL de PBS 1X. Les cellules sont ensuite incubées 30 min (37°C) dans du milieu de culture contenant la sonde (5µM). Avant la mesure de fluorescence par cytométrie (Excitation : 488 nm/Emission : 530 nm), les cellules sont rincées dans 4 mL de PBS 1X, centrifugées et reprises dans 0,5 mL de PBS.

### Détection de l'apoptose :

### Test Annexine V/iodure de propidium :

Un des marqueurs de l'apoptose est la translocation du phosphatidyl-serine de la face interne vers la face externe de la membrane plasmique (translocation permettant aux corps apoptotiques d'êtres reconnus et phagocytés par les macrophages).

Le test AV/PI permet de détecter cet événement, grâce à l'affinité de la protéine annexine V pour le phosphatidyl-serine. L'iodure de propidium qui cible l'ADN, permet la discrimination des cellules apoptotiques précoces, des cellules en apoptose tardives et/ou en nécrose.

Après le temps de traitement désiré, le milieu est éliminé, les cellules sont récoltées (par trypsinisation), centrifugées et rincées dans 4 mL de PBS 1X. Le marquage est réalisé en suivant les instructions du fabriquant (Kit Vybrant. Molecular Probe). Rapidement, les cellules sont reprises dans 100 µL de tampon de marquage, dans lequel est ajouté 6µL d'Annexine 5, et 3µg/mL d'iodure de propidium par échantillon. Les cellules sont ensuite incubées 30 min à l'obscurité (à température ambiante). Quatre cent µL de tampon de marquage sont additionnés avant de mesurer la fluorescence de l'annexine 5 (à 530 nm) et de l'iodure de propidium (à 610 nm).

### Activité caspase :

L'activité caspase est un second marqueur de l'apoptose. La famille des caspase regroupe des protéines principalement impliquées dans la régulation de la mort cellulaire par apoptose. L'activité caspase est détectée à l'aide d'un substrat VAD (pour valine-alanine-acide aspartique) couplé à un fluorochrome (FITC. Emission : 530 nm). La détection de cette activité est réalisée en suivant les instructions du kit CasPACE TM (Promega). Après traitement les cellules sont récoltées (par trypsinisation), centrifugées et rincées dans 4ml de PBS 1X. Puis les cellules sont incubées 20 min dans du milieu de culture contenant le réactif (10µM), 20 min à l'obscurité et à température ambiante. Les cellules sont ensuite rincées 2 fois dans 4 mL de PBS 1X avant d'être fixées 30 min dans une solution de formalin. Avant la mesure de fluorescence par cytométrie en flux, les cellules sont rincées 3 fois 5 min) dans du PBS 1X puis reprise dans 0,5 mL de PBS.

### Perméabilisation mitochondriale :

La perméabilisation des pores mitochondriaux est également un marqueur précoce de l'apoptose. Une sonde fluorescente (TMRM : TétraMéthyl Rhodamine Méthyl ester) ; Molecular Probe) se localisant dans la mitochondrie, va diminuer en intensité lorsque celle-ci migre dans le cytoplasme, à la suite de l'ouverture des pores mitochondriaux. Une chute de fluorescence est donc mesurée par cytométrie en flux.

Après 4h de traitement, les cellules sont trypsinées et rincées dans du PBS 1X avant de les incuber 30 min à 37°C dans du milieu de culture contenant la sonde fluorescente (50 nM finale). Après cette incubation, les cellules sont rincées 1 fois 5 min dans du PBS 1X puis reprisent dans 0,5 mL de PBS avant la mesure par cytométrie.

### Exemple 4 : Gamme de concentration en HE : cytotoxicité ciblée

Les gammes de concentrations en HE (en % d'HE par volume de milieu de culture) pour lesquelles on observe la propriété de cytotoxicité préférentiellement induite dans les cellules mutées (HaCat et A431) par rapport aux cellules non mutées (HEK001 et NHEK894) sont :
de 0.0125% à 0.0175% d'HE d'Origan.
de 0.035% à 0.045% d'HE de Bois de rose

Dans ces gammes de concentrations, les taux de toxicité sont deux fois élevés dans les cellules mutées que dans les cellules non mutées où la viabilité reste de l'ordre de 60%. Par ailleurs, il est à souligner que des études ont montré une bonne pénétration cutanée des principaux ingrédients des HE de Bois de rose et d'Origan.

### Références

Bakkali, F., et al. Antigenotoxic effects of three essential oils in diploid yeast (Saccharomyces cerevisiae) after treatments with UVC radiation, 8-MOP plus UVA and MMS. Mutat.Res. 606: 27-38, 2006**.**
Calcabrini, A., et al. Terpinen-4-ol, the main component of Melaleuca alternifolia (tea tree) oil inhibits the in vitro growth of human melanoma cells. J. Invest. Dermatol. 122: 349-360, 2004.
Diaz, C., et al. Chemical composition of Schinus molle essential oil and its cytotoxic activity on tumour cell lines. Nat. Prod. Res. 22: 1521-1534, 2008**.**
Fusenig, N. E. and Boukamp, P. Multiple stages and genetic altérations in immortalization, malignant transformation, and tumor progression of human skin keratinocytes. Mol. Carcinog. 23: 144-158, 1998**.**
Gogvadze V. et al. Mitochondria in cancer cells: what is so spécial about them ? Trends Cell Biol. 18: 165-173, 2008**.**
Itharat, A., et al. In vitro cytotoxic activity of Thai medicinal plants used traditionally to treat cancer. J.Ethnopharmacol. 90: 33-38, 2004**.**
Kaur, M., et al. Skin cancer chemopreventive agent, a-santalol, induces apoptotic death of human epidermoid carcinoma A431 cells via caspase activation together with dissipation of mitochondrial membrane potential and cytochrome c release. Carcinogenesis 26: 369-380, 2005**.**
Koba K., et al. In vitro cytotoxic activity of Cymbopogon citratus L. and Cymbopogon nardus L. essential oils from Togo. Bangladesh J.Pharmacol. 4: 29-34, 2009**.**
Kumar, A., et al. An essential oil and its major constituent isointermedeol induce apoptosis by increased expression of mitochondrial cytochrome c and apical death receptors in human leukaemia HL-60 cells. Chem. Biol. Interact. 171: 332-347, 2008**.**
Loizzo, M. et al. Antiproliferative effects of essential oils and their major constituents in human renal adenocarcinoma and amelanotic melanoma cells. Cell Prolif. 41: 1002-1012, 2008**.**
Molassiotis, A., et al. Use of complementary and alternative medicine in cancer patients: a European survey. Ann. Oncol. 16: 655-663, 2005**.**
Mudgil, A. V., Segal, N., Andriani, F., Wang, Y., Fusenig, N. E., and Garlick, J. A. Ultraviolet B irradiation induces expansion of intraepithelial tumor cells in a tissue model of early cancer progression. J. Invest. Dermatol. 121: 191-197, 2003**.**
Ortonne, J. P. From actinie keratosis to squamous cell carcinoma. Br. J. Dermatol. 146 Suppl 61: 20-23, 2002**.**
Sharma, P. R., et al. Anticancer activity of an essential oil from Cymbopogon flexuosus. Chem. Biol. Interact. 2008**.**
Taguchi, M., et al. Aberrations of the tumor suppressor p53 gene and p53 protein in solar keratosis in human skin. J. Invest. Dermatol. 103: 500-503, 1994**.**
Verma, M., et al. Induction of mitochondrial-dependent apoptosis by an essential oil from Tanacetum gracile. Planta Med. 74: 515-520, 2008**.**
Wischermann, K., et al. UVA radiation causes DNA strand breaks, chromosomal aberrations and tumorigenic transformation in HaCaT skin keratinocytes. Oncogene 27: 4269-4280, 2008**.**

## Revendications

1. Composition comprenant une huile essentielle extraite de plantes de l'espèce *Origanum compactum* pour une utilisation chez un être humain dans le traitement ciblé de kératoses actiniques dépourvues de cellules tumorales, lesdites kératoses **se caractérisant par** des cellules porteuses de mutations dans le gène p53.

2. Composition pour une utilisation selon la revendication 1 où ladite huile essentielle est appliquée en combinaison avec d'autres composés, notamment en association avec des huiles végétales.

3. Composition pour une utilisation selon l'une quelconque des revendications 1 à 2 pour une application topique.

4. Composition pour une utilisation selon l'une quelconque des revendications 1 à 3, induisant une cytotoxicité préférentielle dans les kératinocytes hyperprolifératifs par rapport aux kératinocytes non-hyperprolifératifs.

5. Composition pour une utilisation selon l'une quelconque des revendications 1 à 4, où l'huile essentielle constitue un principe actif de la composition.

## Patentansprüche

1. Zusammensetzung ein ätherisches Öl umfassend, das durch Extraktion aus Pflanzen der Gattung *Orignaum compactum* gewonnen wird, zur Verwendung beim Menschen zur gezielten Behandlung von tumorzellenfreien aktinischen Keratosen, wobei die Keratosen durch Zellen gekennzeichnet sind, die Träger von Mutationen im p53-Gen sind.

2. Zusammensetzung zur Verwendung nach Anspruch 1,
bei der das ätherische Öl in Kombination mit anderen Verbindungen angewandt wird, insbesondere zusammen mit pflanzlichen Ölen.

3. Zusammensetzung zur Verwendung nach einem der Ansprüche 1 bis 2,
für eine topische Anwendung.

4. Zusammensetzung zur Verwendung nach einem der Ansprüche 1 bis 3,
die in Bezug auf nicht hyperproliferative Keratinozyten eine bevorzugte Zytotoxizität in den hyperproliferativen Keratinozyten bewirkt.

5. Zusammensetzung zur Verwendung nach einem der Ansprüche 1 bis 4,
bei der das ätherische Öl einen aktiven Wirkstoff der Zusammensetzung bildet.

## Claims

1. A composition comprising an essential oil extracted from plants of the species *Origanum compactum,* for use in a human being in the targeted treatment of actinic keratoses, which are free of tumor cells, wherein said keratoses comprise cells carrying mutations in the p53 gene.

2. The composition for use according to claim 1, wherein said essential oil is applied in combination with other compounds, in particular in association with vegetable oils.

3. The composition for use according to any one of claims 1 -2, wherein said composition is topically applied.

4. The composition for use according to any one of claims 1-3, inducing a preferential cytotoxicity in hyperproliferative keratinocytes compared with non-hyperproliferative keratinocytes.

5. The composition for use according to any one of claims 1-4, wherein the essential oil constitutes an active principle of the composition.
